# EUROPÄISCHE PATENTSCHRIFT

(11) **EP 0 527 365 B1**
(45) Veröffentlichungstag und Bekanntmachung des Hinweises auf die Patenterteilung: **08.01.1997**
(21) Anmeldenummer: 92112535.7
(22) Anmeldetag: 22.07.1992
(51) Int. Cl.: C07D 209/48, C08G 73/14, C08G 73/16

(54) **Benzophenoniminodiimide und daraus abgeleitete thermostabile Polymere**
Benzophenoniminodiimides and thermostable polymers derived therefrom
Benzophénoniminodiimides et des polymères thermostables qui en dérivents

(30) Priorität: 13.08.1991 AT 1590/91
(43) Veröffentlichungstag der Anmeldung: 17.02.1993
(73) Patentinhaber: DSM Chemie Linz GmbH, 4021 Linz (AT)
(72) Erfinder: Greber, Gerd, Dr., A-2540 Bad Vöslau (AT); Gruber, Heinrich, Dr., A-1190 Wien (AT); Hassanein, Afschin, Dr., A-1030 Wien (AT)
(74) Vertreter: Kunz, Ekkehard, Dr.

(56) Entgegenhaltungen:
- EP-A- 0 249 262
- GB-A- 2 035 295

## Beschreibung

Die Erfindung betrifft Benzophenoniminodiimide und daraus abgeleitete thermostabile Polymere.

Die bis heute technisch wichtigsten thermostabilen Polymere sind vollaromatische Polyimide, Polyamide und Polyamidimide. Polyimide weisen zwar eine extrem hohe Thermostabilität auf, sind aber meist weder schmelzbar noch löslich und daher nur aufwendig und teuer zu verarbeiten. Die etwas weniger thermostabilen aromatischen Polyamide sind ebenfalls unschmelzbar und in den meisten organischen Lösungsmitteln unlöslich oder nur wenig löslich, so daß ihre Verarbeitung aus Lösungen zu Fasern oder Folien mit großen Schwierigkeiten verbunden ist.

Durch Einbau von Diaminen oder Dianhydriden mit flexiblen Kettenelementen (-CH₂-, -O-, -S-, -CO-) oder aber von sterisch anspruchsvollen Diaminen, wie in EP-A-0 162 606 und US-PS 3,895,064 beschrieben, wird zwar die Löslichkeit und Verarbeitbarkeit von aromatischen Polymeren verbessert, jedoch die Thermostabilität deutlich verringert.

Aus GB 2 035 295 sind Iminodiimide von 3,3',4,4'-Benzophenontetracarbonsäuredianhydrid bekannt, die als UV-Stabilisatoren in Schmiermitteln verwendet werden.

Es wurden nun neue Monomere auf der Basis von 3,3',4,4'-Benzophenontetracarbonsäuredianhydrid (BTDA) durch Einbau von Azomethingruppen gefunden, die zu Polymeren mit hoher Thermostabilität, guter Löslichkeit, niedrigen Glastemperaturen und einfacher thermoplastischer Verarbeitbarkeit führen.

Gegenstand der vorliegenden Erfindung sind demnach Benzophenoniminodiimide der Formel I 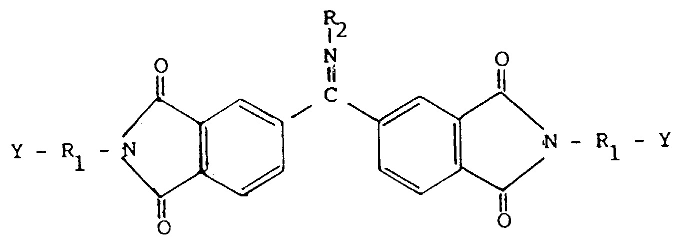 in der Y Hydroxyl oder Carboxyl,
R₁ einen geradkettigen oder verzweigten Alkylenrest mit 1 bis 6 C-Atomen oder einen gegebenenfalls mit Cl, (C₁ bis C₅)-Alkyl oder (C₁ bis C₅)-Alkoxy substituierten o-, m- oder p-Phenylen- oder Benzylidenrest, und
R₂ den Rest OH, einen geradkettigen oder verzweigten, gesättigten oder ein- oder mehrfach ungesättigten Alkylrest mit 1 bis 20 C-Atomen, der mit Cl, OH, NH₂, CONH₂ oder COOR₃, substituiert sein kann, oder einen Rest der Formel NH-CO-NHR₃, worin R₃ Wasserstoff, ein Alkylrest mit 1 bis 4 C-Atomen oder ein Phenyl- oder Benzylrest sein kann,
bedeuten.

In der Formel I bedeuten R₁ einen geradkettigen oder verzweigten Alkylenrest mit 1 bis 6 C-Atomen, wie z.B. einen Methylen-, Ethylen-, Propylen-, Isopropylen-, Butylen-, sek. Butylen- oder Hexylenrest, oder einen unsubstituierten oder mit Cl, Alkylgruppen mit 1 bis 5 C-Atomen, wie z.B. Methyl-, Ethyl- oder Propylgruppen, oder Alkoxygruppen mit 1 bis 5 C-Atomen, wie z.B. Methoxy-, Ethoxy- oder Propoxygruppen, substituierten o-, m- oder p-Phenylen- oder Benzylidenrest, und R₂ den Rest OH, oder einen geradkettigen oder verzweigten, gesättigten oder ein- oder mehrfach ungesättigten, unsubstituierten oder mit OH, Cl, NH₂, CONH₂ oder COOR₃ substituierten Alkylrest mit 1 bis 20 C-Atomen, wie z.B. ein Methyl-, Ethyl-, Hexyl-, Dodecyl-, Oktadecyl-, Vinyl-, Allyl-, Butadienyl-, Isoprenyl-, Hydroxyethyl-, Hydroxyhexyl-, Hydroxydodecyl-, Hydroxyoktadecyl-, Carboxymethyl-, Carboxyethyl-, Carboxydodecyl- Carboxyoktadecyl-, Carboxyhexadecenyl-, Aminohexyl-, Aminododecyl- oder Aminotridecenylrest, oder einen Rest der Formel -NH-CO-NHR₃, wie z.B. Semicarbazyl, Methylsemicarbazyl, Ethylsemicarbazyl, Phenylsemicarbazyl oder Benzylsemicarbazyl.

R₃ bedeutet Wasserstoff oder einen Alkylrest mit 1 bis 4 C-Atomen, wie z.B. einen Methyl-, Ethyl- oder Propylrest.

Bevorzugt sind Verbindungen der Formel I, in der R₁ einen geradkettigen Alkylenrest mit 1 bis 4 C-Atomen, wie z.B. einen Methylen-, Ethylen- oder Butylenrest, oder einen unsubstituierten oder mit Alkyl- oder Alkoxygruppen mit 1 bis 4 C-Atomen, wie z.B. Methyl-, Ethyl-, Propyl-, Methoxy-, Ethoxy- oder Propoxygruppen, substituierten m- oder p-Phenylen- oder Benzylidenrest und R₂ den Rest OH, oder einen geradkettigen, gesättigten, unsubstituierten oder mit OH, NH₂ oder COOH substituierten Alkylrest mit 1 bis 18 C-Atomen, wie z.B. einen Methyl-, Ethyl-, Hexyl-, Dodecyl- Oktadecyl-, Hydroxyethyl-, Hydroxyhexyl-, Carboxymethyl-, Carboxyethyl-, Carboxyoktadecyl-, Aminoethyl-, oder Aminohexylrest, oder einen Semicarbazyl- oder Phenylsemicarbazylrest bedeuten.

Besonders bevorzugt sind dabei Verbindungen der Formel I, in der R₁ einen Ethylen- oder unsubstituierten p-Phenylenrest und R₂ den Rest OH, oder einen geradkettigen, gesättigten unsubstituierten oder mit OH, NH₂ oder COOH substituierten Alkylrest mit 1 bis 8 C-Atomen, wie z.B. einen Methyl-, Ethyl-, Propyl-, Hexyl-, Hydroxyethyl-, Hydroxyhexyl-, Carboxymethyl-, Carboxyethyl-, Aminomethyl-, Aminoethyl- oder Aminohexylrest, oder einen Semicarbazyl- oder Phenylsemicarbazylrest bedeuten.

Ein weiterer Gegenstand der vorliegenden Erfindung ist ein Verfahren zur Herstellung von Verbindungen der Formel I 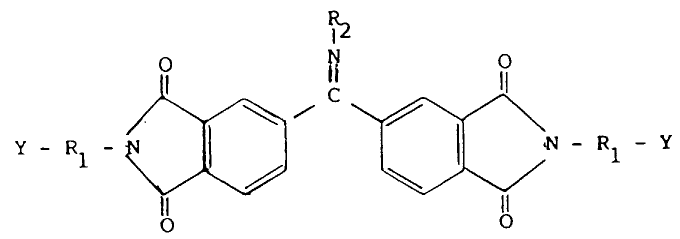 in der Y, R₁, R₂,und R₃ wie oben definiert sind, das dadurch gekennzeichnet ist, daß Verbindungen der Formel II 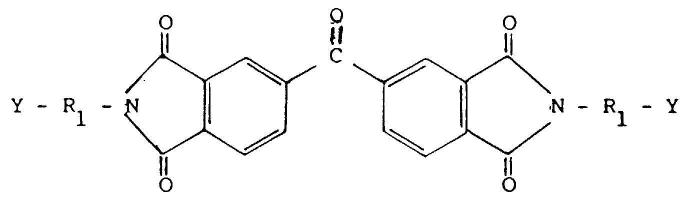 in der R₁ und Y wie oben definiert sind, mit Verbindungen der Formel III

R₂-NH₂

in der R₂ wie oben definiert ist, umgesetzt werden.

Die als Ausgangsverbindungen eingesetzten Benzophenontetracarbonsäurediimid (BTDI)-derivate der Formel II können, wie beispielsweise in Mosher W.A.K., Chlystek, S., J.Heterocycl. Chem. Band 9, (1972) S 319 angegeben, durch Umsetzung von BTDA mit einer äquimolaren Menge eines Aminoalkohols oder einer Aminocarbonsäure, wie etwa Ethanolamin, p-Aminophenol, p-Aminobenzoesäure, hergestellt werden.

Die BTDI-derivate werden dann mit Aminen der Formel III zu den gewünschten Benzophenoniminodiimiden (Schiff`schen Basen) umgesetzt.

Pro Mol BTDI-derivat werden 1 bis 10 Mole, vorzugsweise 1.05 bis 5 Mole an Amin der Formel III eingesetzt.

Amine der Formel III sind beispielsweise n-Hexylamin, Hydrazin, Glycin, Hydroxylamin, Ethanolamin, Semicarbazid, Phenylsemicarbazid, Hexamethylendiamin, Aminopalmitinsäure oder Aminostearinsäure.

Die Umsetzung erfolgt vorzugsweise in einem unter Reaktionsbedingungen inerten Verdünnungsmittel, wie z.B. Dimethylacetamid (DMA), Dimethylformamid (DMF), N-Methylpyrrolidon (NMP), Dimethylsulfoxid (DMSO), Tetramethylharnstoff oder Hexamethylphosphorsäuretriamid (HMP).

Bevorzugt wird DMA als Verdünnungsmittel eingesetzt. Zu dem Reaktionsgemisch aus BTDI-derivat und Amin können geringe Mengen, etwa 0.01 Mol eines Katalysators, wie z.B. Toluolsulfonsäure, Essigsäure, eine Mineralsäure oder ein saurer Ionenaustauscher zugegeben werden. Zweckmäßig wird das bei der Reaktion gebildete Wasser durch azeotrope Destillation mit einem unter Reaktionsbedingungen inerten Lösungsmittel, wie z.B. Benzol oder Toluol, entfernt. Das überschüssige Amin kann gegebenenfalls durch Destillation oder Umkristallisation bzw. auch als Salz rückgewonnen werden. Es können auch Salze der Anine, wie z.B. Hydroxylaminhydrochlorid eingesetzt werden. Als Verdünnungsmittel können auch DMA/Wasser-Gemische verwendet werden, aus denen die gewünschte Verbindung dann ausgefällt wird. Die Isolierung der erfindungsgemäßen Schiff'schen Basen kann je nach deren physikalischen Eigenschaften durch übliche Methoden, wie z.B. Kristallisation, Extraktion oder Ausfällen, erfolgen.

Die so isolierten Rohprodukte können, falls erforderlich, anschließend noch durch bekannte Methoden, wie z.B. Umkristallisieren, Umfällen oder Chromatographie gereinigt werden. Die erfindungsgemäßen Schiff`schen Basen können dann zur Herstellung von Polymeren verwendet werden.

Weitere Gegenstände der vorliegenden Erfindung sind demnach Polyester der Formel IVa

[CO-Z-CO-O-Ar-O]

oder IVb

[O-Z-O-CO-Ar-CO]

in der Z einen substituierten oder unsubstituierten, geradkettigen oder verzweigten Alkylenrest mit 1 bis 8 C-Atomen, einen o-, m- oder p-Phenylenrest oder einen Rest der Formel V 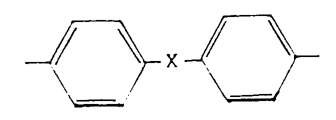 worin X -CH₂-, -C(CH₃)₂-, -O-, -S-, oder -SO₂- sein kann und Ar einen Rest der Formel VI 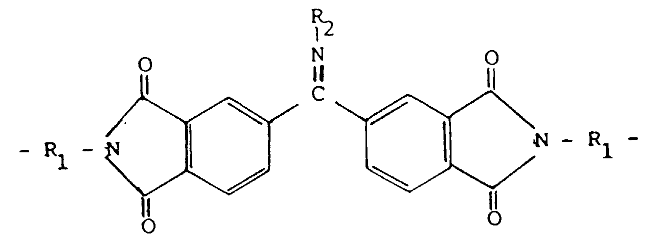 in der R₁ und R₂ wie oben definiert sind, bedeuten, und Polyamide der Formel VII

⁅NH-Z-NH-CO-Ar-CO⁆

in der Ar und Z wie oben definiert sind.

Bevorzugt sind dabei Polyester der Formel IVa oder IVb in denen Z einen m- oder p-Phenylenrest bedeutet und R₁, R₂ und R₃ wie oben definiert sind.

Bevorzugte Polyamide sind Polyamide der Formel VII in der Z einen Rest der Formel VIII 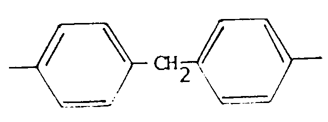 bedeuted und R₁, R₂ und R₃ wie oben definiert sind.

Die Herstellung der Polymeren kann durch Lösungspolykondensation in einem unter Reaktionsbedingungen inerten Verdünnungsmittel, wie etwa DMA, HMP, NMP, erfolgen.

Polyester der Formel IVa werden aus den erfindungsgemäßen Schiff`schen Basen der Formel I, in der Y Hydroxyl bedeutet, durch Umsetzung mit äquimolaren Mengen an Dicarbonsäuredichloriden, wie z.B. Isophthalsäuredichlorid (IPDCL), Terephthalsäuredichlorid (TPDCL) hergestellt.

Zur Herstellung von Polyestern der Formel IVb aus Schiff'schen Basen der Formel I, in der Y Carboxyl bedeutet, muß die Carboxylgruppe aktiviert werden, beispielsweise durch Umwandlung in die entsprechenden Dicarbonsäuredichloride, -anhydride oder -ester. Aus den Dicarbonsäuredichloriden werden dann durch Umsetzung mit Diolen, wie z.B. Ethylenglykol, Hydrochinon oder Bisphenol-A die gewünschten Polyester der Formel IVb hergestellt.

Zur Herstellung der Polyamide der Formel VII werden ebenfalls die Schiff`schen Basen der Formel I, in der Y Carboxyl bedeutet, durch Reaktion mit Thionylchlorid in die entsprechenden Dicarbonsäuredichloride überführt und dann mit einer äquimolaren Menge eines Diamins, wie etwa Bis-(4-Aminophenyl)methan (MDA), umgesetzt.

In den Fällen, in denen das Dicarbonsäuredichlorid mit einem Diol oder einem Diamin umgesetzt wird, ist es notwendig, die entstehende Salzsäure zu entfernen. Dies geschieht vorteilhafterweise durch Zugabe einer äquivalenten Mengen einer geeigneten Base, wie z.B. Triethylamin, Pyridin oder Dimethylanilin.

Die Reaktionstemperatur liegt je nach verwendeter Schiff`schen Base und gewünschtem Polymer zwischen -30 °C und 50 °C.

Gegebenenfalls wird die Reaktion unter Stickstoffatmosphäre durchgeführt.

Die Isolierung der Polymere kann durch übliche Methoden, z.B. durch Ausfällen mit Wasser oder anderen geeigneten Verdünnungsmitteln erfolgen.

Die Polyester und Polyamide zeichnen sich durch sehr hohe Thermostabilität und niedrige Glastemperatur aus, wodurch eine thermoplastische Verarbeitung ermöglicht wird. Die Herstellung von Folien und Fasern wird durch die ausgezeichnete Löslichkeit in DMA erleichtert.

### Beispiel 1

### a) Herstellung von N,N'-Bis(Hydroxyethyl)-3,3',4,4'-Benzophenontetracarbonsäurediimid

In einem Dreihalskolben mit Magnetrührer, Tropftrichter und Thermometer wurde 20 g (0,062 Mol) BTDA vorgelegt, in 180 ml DMA gelöst und aus einem Tropftrichter 7,58 g (0,124 Mol) Ethanolamin gelöst in 20 ml DMA langsam hinzugetropft. Das Reaktionsgemisch wurde dann eine halbe Stunde bei Raumtemperatur gerührt und danach 2 Stunden auf Rückfluß erhitzt.

Zur Aufarbeitung wurde das Reaktionsgemisch nach dem Abkühlen auf Raumtemperatur auf 2 1 Wasser gegossen. Die ausgefallenen Kristalle wurden dann abgesaugt, aus DMF/Benzol = 1:1 umkristallisiert und im Vakuumtrockenschrank bei 80 °C bis zur Gewichtskonstanz getrocknet.
- Ausbeute:: 22,79 g (90 % d.Th.)
- Schmelzpunkt:: 208 - 209 °C

### b) Herstellung von N,N'-Bis(hydroxyphenyl)-3,3',4,4'-Benzophenontetracarbonsäurediimid

Die Reaktionsdurchführung folgte analog zu Beispiel la), wobei das Produkt bereits nach Beginn der Erhitzungsphase ausfiel. Die gelben Kristalle wurden abgesaugt und nach Umkristallisation aus DMF/Benzol = 1:1 im Vakuumtrockenschrank bei 80 °C bis zur Gewichtskonstanz getrocknet.
- Ausbeute:: 27,21 g (87 % d.Th.) gelbe Kristalle
- Schmelzpunkt:: 441 °C

### Beispiel 2

### a) Herstellung der Schiff'schen Base aus N,N'-Bis(Hydroxyethyl)-3,3',4,4'-Benzophenontetracarbonsäurediimid und n-Hexylamin

20 g (0,049 Mol) N,N'-Bis(hydroxyethyl)-3,3',4,4'-benzophenontetracarbonsäurediimid wurden in der Hitze in 150 ml abs. Dimethylacetamid (DMA) gelöst.und 24,78 g (0,245 Mol) n-Hexylamin, gelöst in 30 ml abs. Toluol als Wasserschlepper, wurden langsam hinzugetropft. Anschließend wurden 2 Spatelspitzen p-Toluolsulfonsäure als Katalysator zum Reaktionsgemisch gegeben und die Temperatur 24 Stunden zwischen 120 °C und 130 °C gehalten.

Danach wurde DMA zusammen mit Toluol und dem gebildeten Wasser im Vakuum abdestilliert, der verbleibende Rückstand in Methylenchlorid aufgenommen und mit Wasser zur Entfernung des Katalysators und von Lösungsmittelresten extraktiv gereinigt. Die organische Phase wurde gesammelt, über Natriumsulfat getrocknet und eingedampft. Das so erhaltene Rohprodukt wurde durch Säulenchromatographie gereinigt.
- Ausbeute:: 13,2 g (55 % d.Th.) gelbe Flüssigkeit (1)

Die Produkte (3) - (7) wurden analog zu Beispiel 2a) hergestellt.

Die Elementaranalysen und Iminbanden sind aus Tabelle 1 ersichtlich.

### b) Herstellung der Schiff'schen Base aus N,N'-Bis(Hydroxyethyl)-3,3',4,4'-Benzophenontetracarbonsäurediimid und Hydroxylamin

15,14 g (0,074 Mol) Hydroxylaminhydrochlorid und 4,8 g (0,06 Mol) Natriumacetat wurden in 30 ml Wasser gelöst und auf 60 °C erwärmt. Danach wurden 20 g (0,049 Mol) N,N'-Bis(hydroxyethyl)-3,3',4,4'-benzophenontetracarbonsäurediimid langsam hinzugetropft. Nach 30 Minuten wurde das Reaktionsgemisch auf 0 °C abgekühlt und das abgeschiedene Oxim abgesaugt. Die gelben Kristalle wurden nach Reinigung mittels Säulenchromatographie bei 60 °C im Vakuumtrockenschrank bis zur Gewichtskonstanz getrocknet.
- Ausbeute:: 12,45 g (60 % d.Th.) gelbe Kristalle (2)
- Schmelzpunkt:: 250 - 253 °C

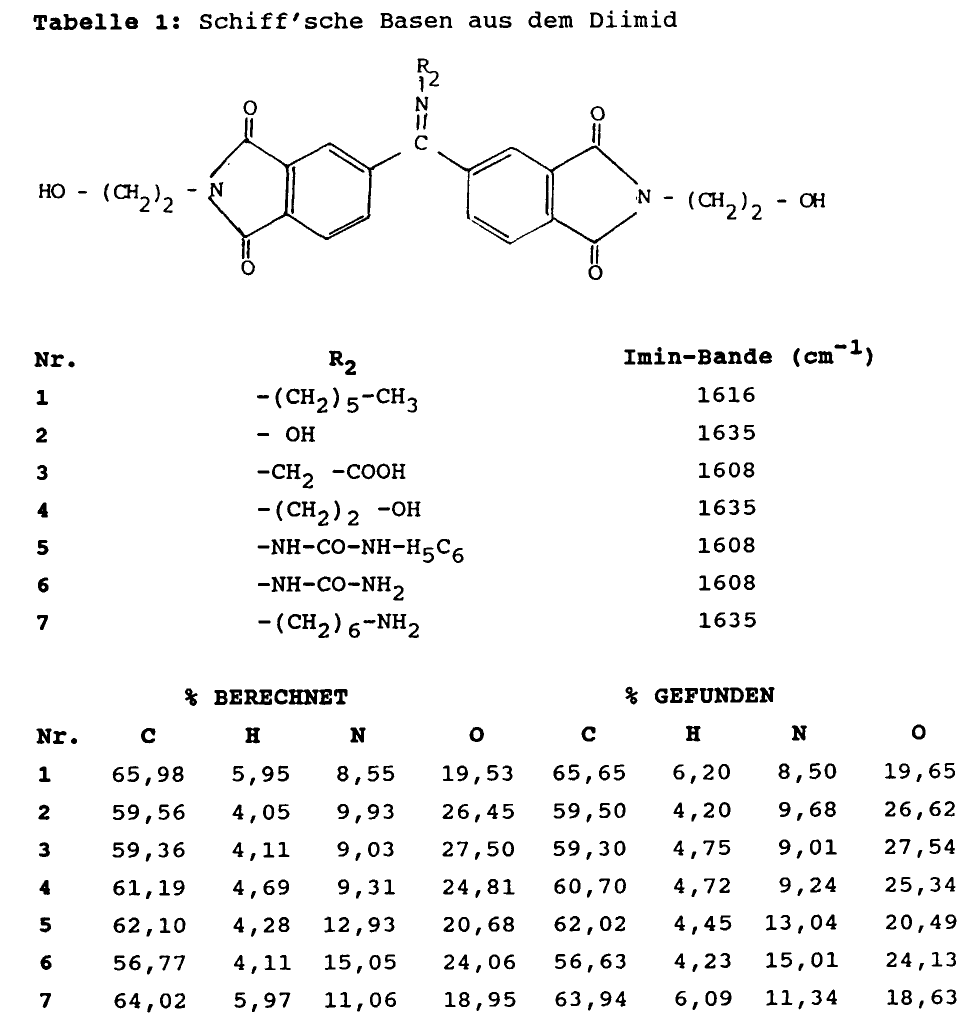

Es wurden eine Reihe von Schiff'schen Basen aus N,N-Bis(hydroxyphenyl)-3,3',4,4'-benzophenontetracarbonsäurediimid und N,N'-Bis(carboxyphenyl)-3,3',4,4'-benzophenontetracarbonsäurediimid analog zu den in Beispiel 2a und 2b beschriebenen Verbindungen hergestellt.

Elementaranalysen und Iminbanden sind aus Tabelle 2 und Tabelle 3 ersichtlich.

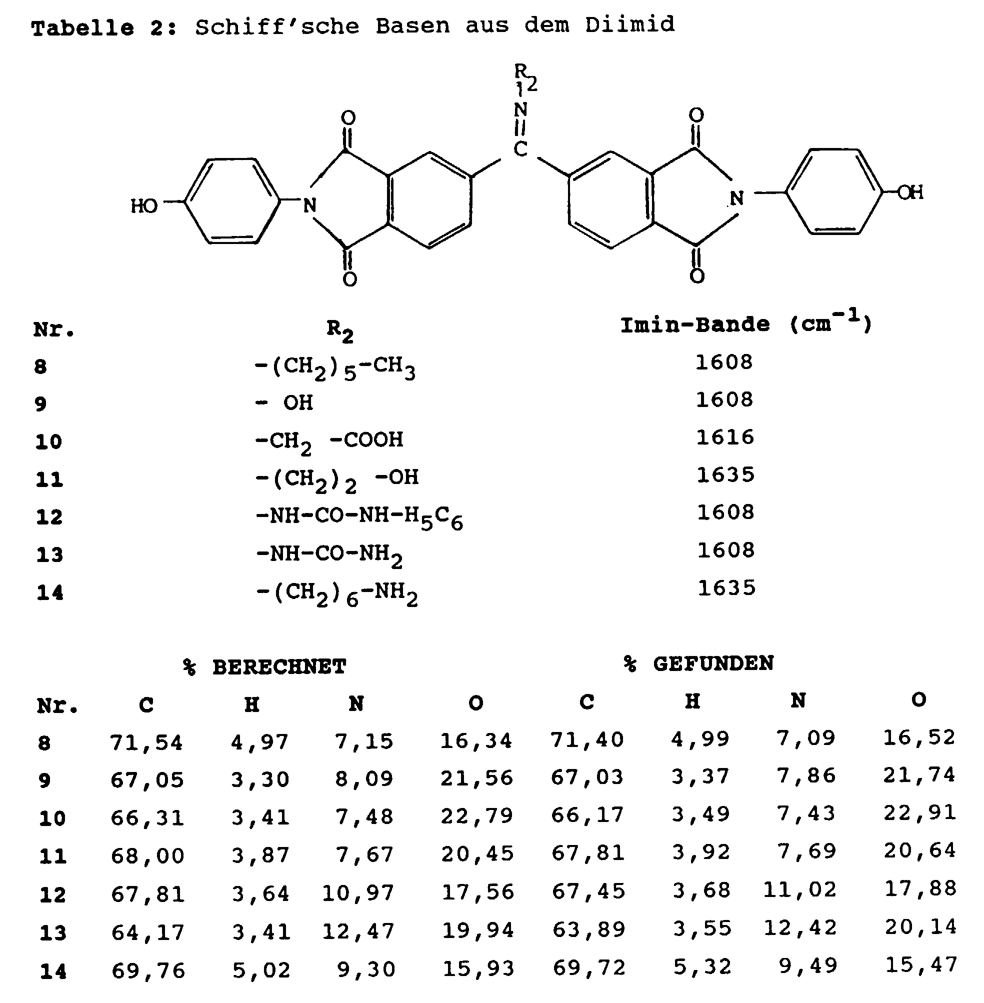

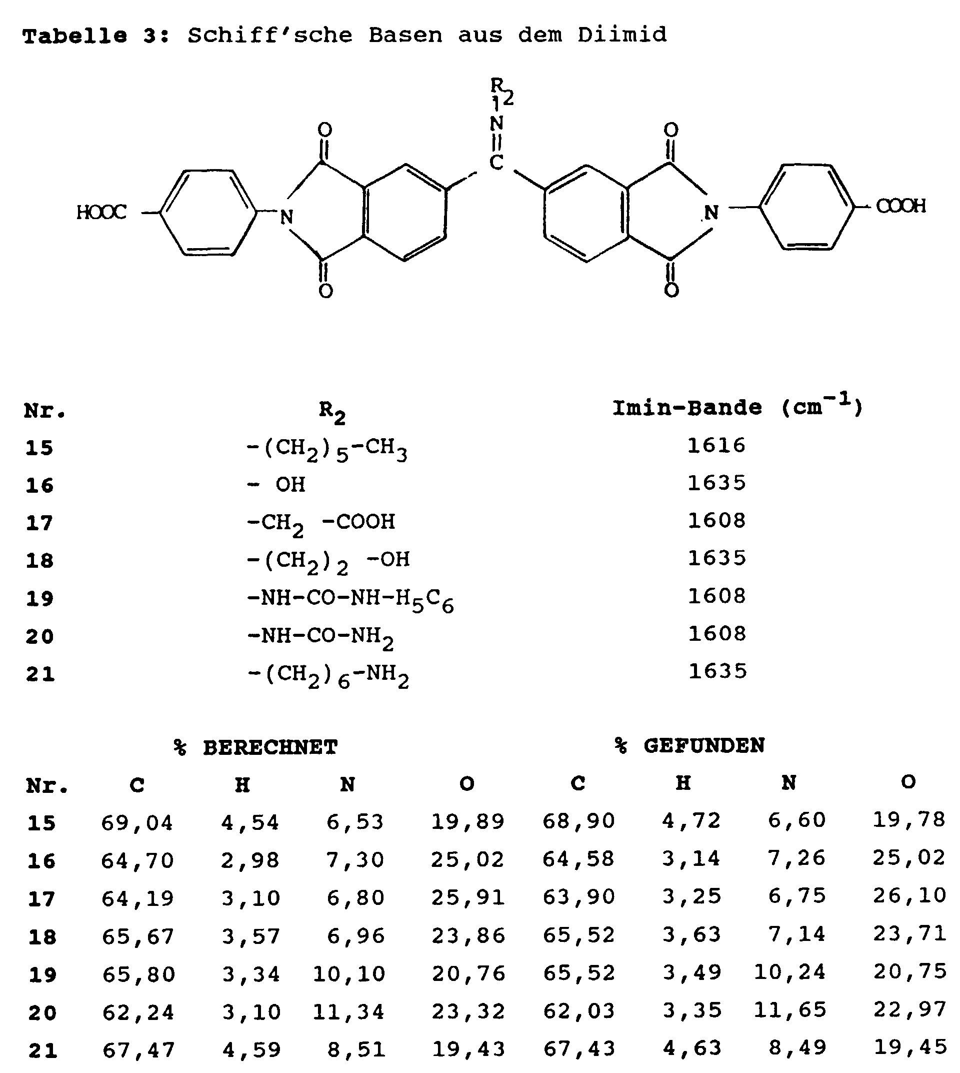

### Beispiel 3

### Herstellung eines Polyesters aus N,N'-Bis(Hydroxyethyl)-3,3',4,4'-Benzophenontetracarbonsäurediimid und Isophthalsäuredichlorid (IPDCL)

10 g (0,025 Mol) N,N'-Bis(hydroxyethyl)-3,3',4,4'-benzophenontetracarbonsäurediimid und 5,08 g (0,025 Mol) IPDCL wurden bei Raumtemperatur in 50 ml abs. DMA gelöst. Zu der auf 0 - 5 °C abgekühlten Reaktionslösung wurden langsam 5,75 g (0,057 Mol) Triethylamin als Säurefänger, gelöst in 10 ml abs. DMA, zugetropft. Nach Zusatz von weiteren 0,5 ml Triethylamin wurde die viskose Lösung 2,5 Stunden bei 20 °C gerührt. Das entstande Salz wurde abfiltriert, das Polymer mit Wasser gefällt, abzentrifugiert, mit Wasser gewaschen und im Vakuumtrockenschrank bei 80 °C bis zur Gewichtskonstanz getrocknet.
- Ausbeute:: 12,52 g (93 % d.Th.) (22)

Eine Reihe anderer Polyester wurden nach derselben Methode hergestellt. Ihre Glastemperaturen, die inhärenten Viskositäten, die Thermostabilitäten, die Löslichkeiten, das Filmbildungsvermögen und die Elementaranalysen sind in Tabelle 4 zusammengefaßt.

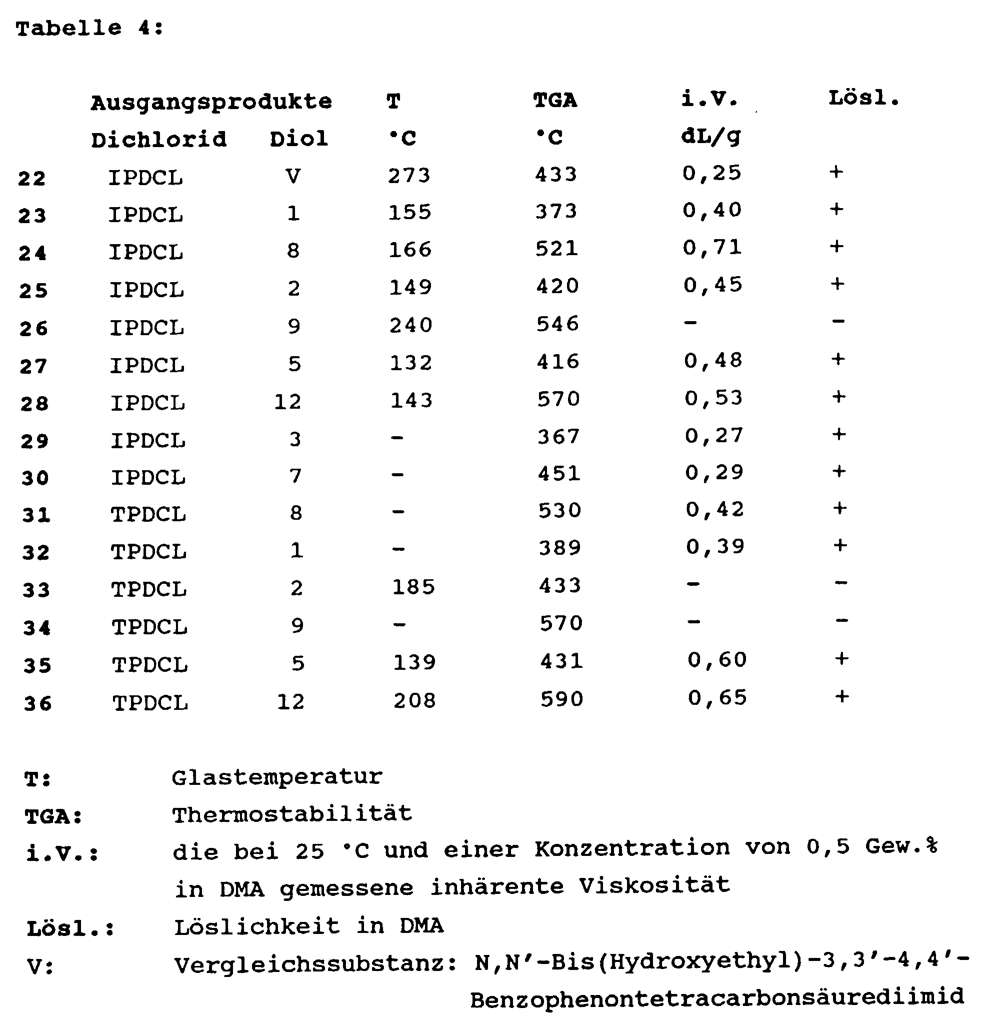

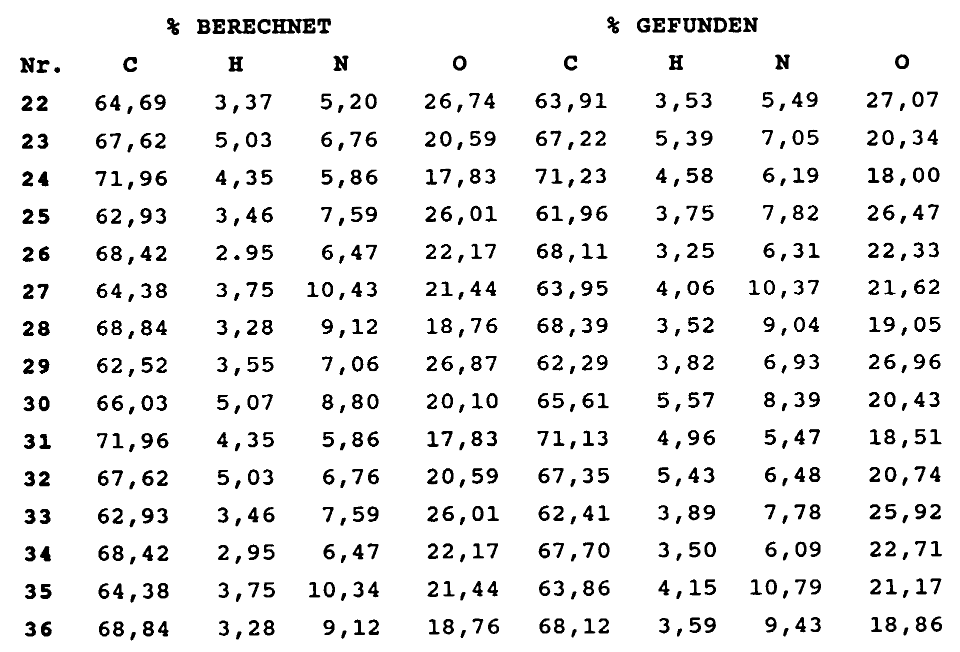

### Beispiel 4

### Herstellung eines Polyamides aus (15) und Bis-(4-Aminophenyl)methan (MDA)

1,9822 g (0,01 Mol) MDA wurden unter Stickstoffatmosphäre in einem Gemisch aus 25 ml abs. N-Methyl-2-pyrrolidon (NMP) und 50 ml abs. Hexamethylphosphorsäuretriamid (HMP) bei Raumtemperatur gelöst. Die Lösung wurde auf -15 °C gekühlt und 15 Minuten stehengelassen. Anschließend wurden 6,8054 g (0,01 Mol) Dicarbonsäuredichlorid (hergestellt aus Dicarbonsäure (15) und Thionylchlorid in abs. Benzol; wird nach Herstellung direkt eingesetzt) auf einmal zugegeben. Das Reaktionsgemisch wurde langsam auf Raumtemperatur erwärmt und anschließend über Nacht unter Stickstoff gerührt. Die entstandene zähflüssige Masse wurde unter Rühren in 500 ml heißes Wasser gegossen, das ausgefallene zerkleinerte Polymer mehrmals mit Wasser gewatrocknet.
- Ausbeute:: 7,49 g (93 % d.Th.) (37)

Alle anderen Polyamide wurden nach derselben Methode hergestellt. Ihre Glastemperaturen, inhärente Viskosität, Thermostabilität, Löslichkeit und Elementaranalyse sind in Tabelle 5 zusammengefaßt.

**Tabelle 5:**

| | **Reaktionspartner** | **T °C** | | **TGA °C** | **i.V. dL/g** | | **Lösl.** | |
|---|---|---|---|---|---|---|---|---|
| **37** | 15, MDA | 156 | | 485 | 0,28 | | + | |
| **38** | 16, MDA | 251 | | 535 | 0,34 | | + | |
| **39** | 19, MDA | 187 | | 563 | 0,30 | | + | |
| | | | | | | | | |
| **T:** Glastemperatur | | | | | | | | |
| **TGA:** Thermostabilität | | | | | | | | |
| **i.V.:** die bei 25 °C und einer Konzentration von 0,5 Gew.% in DMA gemessene inhärente Viskosität | | | | | | | | |
| **Lösl.:** Löslichkeit in DMA | | | | | | | | |
| | | | | | | | | |

| | **% BERECHNET** | | | | **% GEFUNDEN** | | | |
|---|---|---|---|---|---|---|---|---|
| **Nr.** | **C** | **H** | **N** | **O** | **C** | **H** | **N** | **O** |
| **37** | 74,52 | 4,88 | 8,69 | 11,91 | 74,03 | 5,25 | 8,37 | 12,35 |
| **38** | 71,64 | 3,69 | 9,49 | 15,18 | 71,09 | 3,98 | 9,32 | 15,61 |
| **39** | 71,57 | 3,89 | 11,46 | 13,09 | 71,12 | 4,16 | 11,39 | 13,33 |

## Patentansprüche (Patentansprüche für folgende(n) Vertragsstaat(en): AT, BE, CH, DE, DK, FR, GB, GR, IT, LI, LU, NL, PT, SE)

1. Verbindungen der Formel I 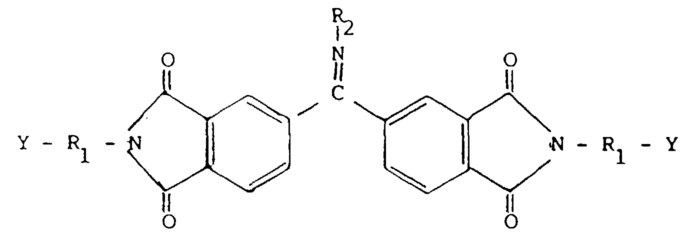 in der Y Hydroxyl oder Carboxyl,
R₁ einen geradkettigen oder verzweigten Alkylenrest mit 1 bis 6 C-Atomen oder einen gegebenenfalls mit Cl, (C₁ bis C₅)-Alkyl oder (C₁ bis C₅)-Alkoxy substituierten o-, m- oder p-Phenylen- oder Benzylidenrest, und
R₂ den Rest OH,einen geradkettigen oder verzweigten, gesättigten oder ein- oder mehrfach ungesättigten Alkylrest mit 1 bis 20 C-Atomen der mit Cl, OH, NH₂, CONH₂ oder COOR₃, substituiert sein kann, oder einen Rest der Formel NH-CO-NHR₃,worin
R₃ Wasserstoff, ein Alkylrest mit 1 bis 4 C-Atomen, ein Phenyl- oder Benzylrest sein kann, bedeuten.

2. Verbindungen der Formel I gemäß Anspruch 1, in der R₁ einen geradkettigen Alkylenrest mit 1 bis 4 C-Atomen oder einen gegebenenfalls mit (C₁ bis C₄)-Alkyl oder (C₁ bis C₄)-Alkoxy substituierten m- oder p-Phenylen- oder Benzylidenrest und R₂ den Rest OH, einen geradkettigen, gesättigten Alkylrest mit 1 bis 18 C-Atomen, der mit OH, NH₂ oder COOH substituiert sein kann, oder einen Rest der Formel NH-CO-NHR₃, worin R₃ Wasserstoff oder ein Phenylrest sein kann. bedeuten.

3. Verbindungen der Formel I gemäß Anspruch I, in der R₁ einen Ethylen- oder p-Phenylenrest und R₂ den Rest OH, einen geradkettigen, gesättigten Alkylrest mit 1 bis 8 C-Atomen, der mit OH, NH₂ oder COOH substituiert sein kann, oder einen Rest der Formel NH-CO-NHR₃, worin R₃ Wasserstoff oder ein Phenylrest sein kann, bedeuten.

4. Verfahren zur Herstellung von Verbindungen der Formel I, dadurch gekennzeichnet, daß Verbindungen der Formel II 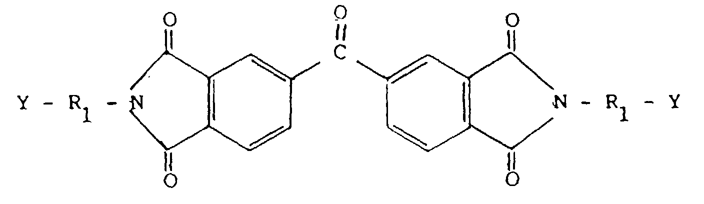 in der R₁ und Y wie in Anspruch 1 definiert sind, mit Verbindungen der Formel III
R₂-NH₂ III
in der R₂ wie im Anspruch 1 definiert ist, umgesetzt werden.

5. Verfahren nach Anspruch 4, dadurch gekennzeichnet, daß 1 bis 10 Mol, bevorzugt 1.05 bis 5 Mol der Verbindung der Formel III pro Mol der Verbindung II eingesetzt werden.

6. Polyester der Formel IVa
⁅CO-Z-CO-O-Ar-O⁆
oder IVb
⁅O-Z-O-CO-AR-CO⁆
in der Z einen substituierten oder unsubstituierten geradkettigen oder verzweigten Alkylenrest mit 1 bis 8 C-Atomen, oder einen o-, m- oder p-Phenylenrest oder einen Rest der Formel V 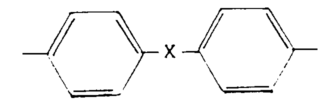 worin X -CH₂-, -C(CH₃)₂-, -O-, -S- oder -SO₂- sein kann, und Ar einen Rest der Formel VI 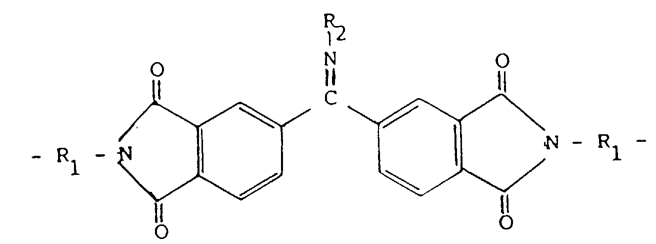 in der R₁ und R₂ wie in Anspruch 1 definiert sind, bedeuten.

7. Polyester der Formel IVa oder IVb gemäß Anspruch 6, in der Z einen m- oder p-Phenylenrest bedeutet und R₁, R₂ und R₃ wie in Anspruch 3 definiert sind.

8. Polyamide der Formel VII
(̵NH-Z-NH-CO-Ar-CO⁆
in der Ar und Z wie in Anspruch 6 definiert sind.

9. Polyamide der Formel VII gemäß Anspruch 7, in der Z einen Rest der Formel VIII 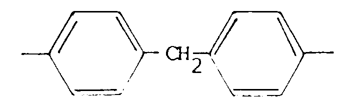 bedeutet und R₁, R₂ und R₃ wie in Anspruch 3 definiert sind.

10. Verwendung der Monomeren der Formel I zur Herstellung von Polymeren.

## Patentansprüche (Patentansprüche für folgende(n) Vertragsstaat(en): ES)

1. Verfahren zur Herstellung von Verbindungen der Formel I 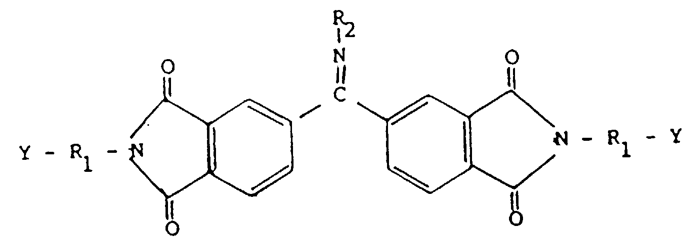 in der Y Hydroxyl oder Carboxyl,
R₁ einen geradkettigen oder verzweigten Alkylenrest mit 1 bis 6 C-Atomen oder einen gegebenenfalls mit Cl, (C₁ bis C₅)-Alkyl oder (C₁ bis C₅)-Alkoxy substituierten o-, m- oder p-Phenylen- oder Benzylidenrest, und
R₂ den Rest OH,einen geradkettigen oder verzweigten, gesättigten oder ein- oder mehrfach ungesättigten Alkylrest mit 1 bis 20 C-Atomen der mit Cl, OH, NH₂, CONH₂ oder COOR₃, substituiert sein kann, oder einen Rest der Formel NH-CO-NHR₃,worin
R₃ Wasserstoff, ein Alkylrest mit 1 bis 4 C-Atomen, ein Phenyl- oder Benzylrest sein kann,
bedeuten, dadurch gekennzeichnet, daß Verbindungen der Formel II 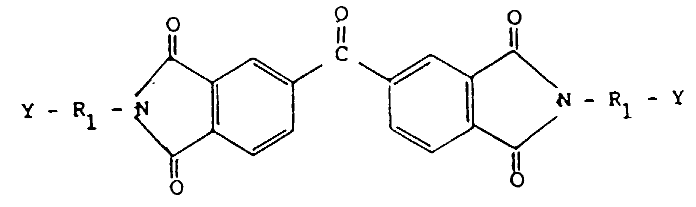 in der R₁ und Y wie oben definiert sind, mit Verbindungen der Formel III
R₂ - NH₂
in der R₂ wie oben definiert ist, in einem unter Reaktionsbedingungen inerten Verdünnungsmittel umgesetzt werden.

2. Verfahren zur Herstellung von Verbindungen der Formel I, in der
R₁ einen geradkettigen Alkylenrest mit 1 bis 4 C-Atomen oder einen gegebenenfalls mit (C₁ bis C₄)-Alkyl oder (C₁ bis C₄)-Alkoxy substituierten m- oder p-Phenylen- oder Benzylidenrest und R₂ den Rest OH, einen geradkettigen, gesättigten Alkylrest mit 1 bis 18 C-Atomen, der mit OH, NH₂ oder COOH substituiert sein kann, oder einen Rest der Formel NH-CO-NHR₃, worin R₃ Wasserstoff oder ein Phenylrest sein kann, bedeuten, dadurch gekennzeichnet, daß Verbindunaen der Formel II 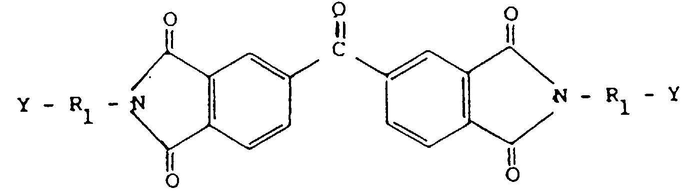 in der R₁ und Y wie oben definiert sind, mit Verbindungen der Formel III
R₂ - NH₂
in der R₂ wie oben definiert ist, in einem unter Reaktionsbedingungen inerten Verdünnungsmittel umgesetzt werden.

3. Verfahren zur Herstellung von Verbindungen der Formel I, in der
R₁ einen Ethylen- oder p-Phenylenrest und R₂ den Rest OH, einen geradkettigen, gesättigten Alkylrest mit 1 bis 8 C-Atomen, der mit OH, NH₂ oder COOH substituiert sein kann, oder einen Rest der Formel NH-CO-NHR₃, worin R₃ Wasserstoff oder ein Phenylrest sein kann, bedeuten, dadurch qekennzeichnet, daß Verbindungen der Formel II 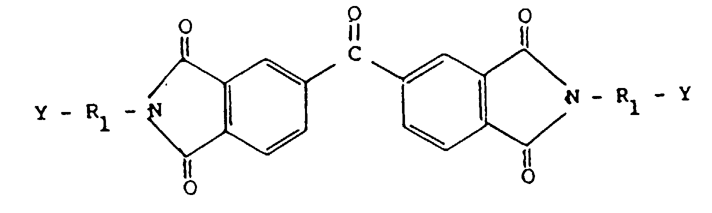 in der R₁ und Y wie oben definiert sind, mit Verbindungen der Formel III
R₂ - NH₂
in der R₂ wie oben definiert ist, in einem unter Reaktionsbedingungen inerten Verdünnungsmittel umgesetzt werden.

4. Verfahren nach Anspruch 1 bis 3, dadurch gekennzeichnet, daß 1 bis 10 Mol, bevorzugt 1,05 bis 5 Mol der Verbindung der Formel III pro Mol der Verbindung II eingesetzt werden.

5. Verfahren zur Herstellung von Polyestern der Formel IVa
⁅CO-Z-CO-O-Ar-O⁆
in der Z einen substituierten oder unsubstituierten geradkettigen oder verzweigten Alkylenrest mit 1 bis 8 C-Atomen, oder einen o-, m- oder p-Phenylenrest oder einen Rest der Formel V 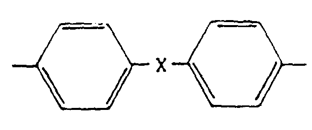 worin X -CH₂-, -C(CH₃)₂-, -O-, -S- oder -SO₂- sein kann, und Ar einen Rest der Formel VI 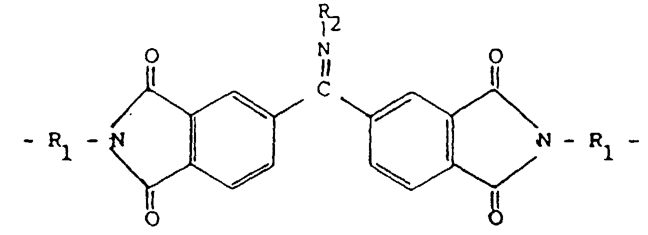 in der R₁ und R₂ wie in Anspruch 1 definiert sind, bedeutet, dadurch gekennzeichnet, daß Verbindungen der Formel I in der Y Hydroxyl bedeutet, und R₁ und R₂ wie in Anspruch 1 definiert sind in einem unter Reaktionsbedingungen inerten Verdünnungsmittel mit Dicarbonsäuredichloriden der Formel VIIa
Cl - CO - Z - CO - Cl
in der Z wie oben definiert ist, bei Temperaturen zwischen -30°C und 50°C umgesetzt werden.

6. Verfahren nach Anspruch 5, dadurch gekennzeichnet, daß Verbindungen der Formel I in der Y Hydroxyl bedeutet und R₁ und R₂ wie in Anspruch 3 definiert sind, mit Dicarbonsäuredichloriden der Formel VIIa in der Z einen m- oder p-Phenylenrest bedeuten und R₁, R₂ und R₃ wie in Anspruch 3 definiert sind, umgesetzt werden.

7. Verfahren zur Herstellung von Polyester der Formel IV b,
⁅O-Z-O-CO-AR-CO⁆
in der Z und Ar wie in Anspruch 5 definiert sind, dadurch gekennzeichnet, daß Verbindungen der Formel I in der Y Carboxyl bedeutet und R₁ und R₂ wie in Anspruch 1 definiert sind, in die entsprechende Dicarbonsäuredichloride, -anhyride oder -ester übergeführt werden und anschließend mit Diolen der Formel VII b
HO - Z - OH
in der Z wie in Anspruch 5 definiert ist, in einem unter Reaktionsbedingungen inerten Verdünnungsmittel, bei Temperaturen von -30°C bis 50°C umgesetzt werden.

8. Verfahren nach Anspruch 7, dadurch gekennzeichnet, daß Verbindungen der Formel I in der Y Carboxyl bedeutet und R₁ und R₂ wie in Anspruch 3 definiert sind, mit Diolen der Formel VIIb in der Z einen m- oder p-Phenylenrest bedeuten und R₁, R₂ und R₃ wie in Anspruch 3 definiert sind, umgesetzt werden.

9. Verfahren zur Herstellung von Polyamiden der Formel VIII
⁅NH-Z-NH-CO-Ar-CO⁆
in der Ar und Z wie in Anspruch 5 definiert sind, dadurch gekennzeichnet, daß Verbindungen der Formel I in der Y Carboxyl bedeutet und R₁ und R₂ wie in Anspruch 1 definiert sind, in das entsprechende Dicarbonsäuredichlorid überführt werden und anschlißend mit einem Diamin der Formel IX
NH₂ - Z - NH₂
in der Z wie in Anspruch 5 definiert ist, in einem unter Reaktionsbedingungen inerten Verdünnungsmittel bei Temperaturen zwischen -30°C und 50°C umgesetzt werden.

10. Verfahren nach Anspruch 9, dadurch gekennzeichnet, daß Verbindungen der Formel I in der Y Carboxyl bedeutet und R₁ und R₂ wie in Anspruch 3 definiert sind, mit Diaminen der Formel IX in der Z einen Rest der Formel X 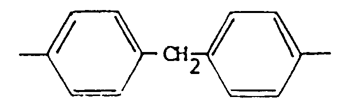 bedeutet und R₁, R₂ und R wie in Anspruch 3 definiert sind, umgesetzt werden.

## Claims (Claims for the following Contracting State(s): AT, BE, CH, DE, DK, FR, GB, GR, IT, LI, LU, NL, PT, SE)

1. Compounds of the formula I 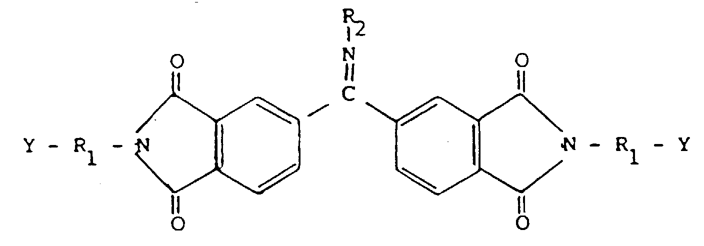 in which Y denotes hydroxyl or carboxyl,
R₁ denotes a straight-chain or branched alkylene radical having 1 to 6 C atoms, or an o-, m- or p-phenylene or benzylidene radical which is optionally substituted by Cl, (C₁ to C₅)-alkyl or (C₁ to C₅)-alkoxy, and
R₂ denotes the radical OH, a straight-chain or branched, saturated or mono- or polyunsaturated alkyl radical having 1 to 20 C atoms, which can be substituted by Cl, OH, NH₂, CONH₂ or COOR₃, or a radical of the formula NH-CO-NHR₃, wherein
R₃ can be hydrogen, an alkyl radical having 1 to 4 C atoms or a phenyl or benzyl radical.

2. Compounds of the formula I according to Claim 1, in which
R₁ denotes a straight-chain alkylene radical having 1 to 4 C atoms or an m- or p-phenylene or benzylidene radical which is optionally substituted by (C₁ to C₄)-alkyl or (C₁ to C₄)-alkoxy, and R₂ denotes the radical OH, a straight-chain, saturated alkyl radical having 1 to 18 C atoms, which can be substituted by OH, NH₂ or COOH, or a radical of the formula NH-CO-NHR₃, wherein R₃ can be hydrogen or a phenyl radical.

3. Compounds of the formula I according to Claim 1, in which
R₁ denotes an ethylene or p-phenylene radical and R₂ denotes the radical OH, a straight-chain, saturated alkyl radical having 1 to 8 C atoms, which can be substituted by OH, N₂ or COOH, or a radical of the formula NH-CO-NHR₃, wherein R₃ can be hydrogen or a phenyl radical.

4. Process for the preparation of compounds of the formula I, characterized in that compounds of the formula II 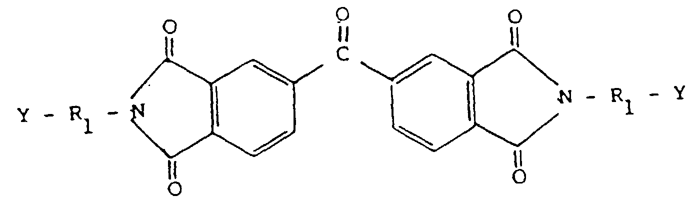 in which R₁ and Y are as defined in Claim 1, are reacted with compounds of the formula III
R₂-NH₂ III
in which R₂ is as defined in Claim 1.

5. Process according to Claim 4, characterized in that 1 to 10 mol, preferably 1.05 to 5 mol of the compound of the formula III are employed per mol of the compound II.

6. Polyesters of the formula IVa
⁅CO-Z-CO-O-Ar-O⁆
or IVb
⁅O-Z-O-CO-Ar-CO⁆
in which Z denotes a substituted or unsubstituted, straight-chain or branched alkylene radical having 1 to 8 C atoms, or an o-, m- or p-phenylene radical or a radical of the formula V 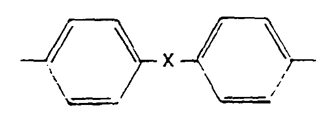 wherein X can be -CH₂-, -C(CH₃)₂-, -O-, -S-, or -SO₂-, and Ar denotes a radical of the formula VI 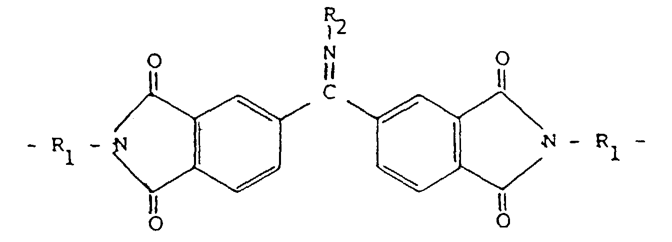 in which R₁ and R₂ are as defined in Claim 1.

7. Polyesters of the formula IVa or IVb according to Claim 6, in which Z denotes an m- or p-phenylene radical and R₁, R₂ and R₃ are as defined in Claim 3.

8. Polyamides of the formula VII
(̵NH-Z-NH-CO-Ar-CO⁆
in which Ar and Z are as defined in Claim 6.

9. Polyamides of the formula VII according to Claim 7, in which Z denotes a radical of the formula VIII 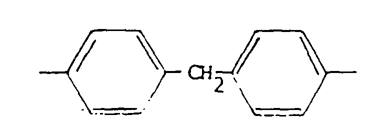 and R₁, R₂ and R₃ are as defined in Claim 3.

10. Use of the monomers of the formula I for the preparation of polymers.

## Claims (Claims for the following Contracting State(s): ES)

1. Process for the preparation of compounds of the formula I 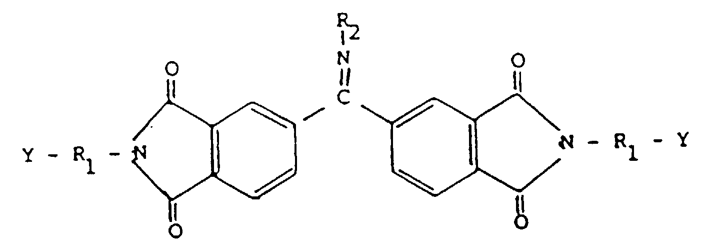 in which Y denotes hydroxyl or carboxyl,
R₁ denotes a straight-chain or branched alkylene radical having 1 to 6 C atoms, or an o-, m- or p-phenylene or benzylidene radical which is optionally substituted by Cl, (C₁-C₅)-alkyl or (C₁-C₅)-alkoxy, and
R₂ denotes the radical OH, a straight-chain or branched, saturated or mono- or polyunsaturated alkyl radical having 1 to 20 C atoms, which can be substituted by Cl, OH, NH₂, CONH₂ or COOR₃, or a radical of the formula NH-CO-NHR₃, wherein
R₃ can be hydrogen, an alkyl radical having 1 to 4 C atoms or a phenyl or benzyl radical, characterized in that compounds of the formula II 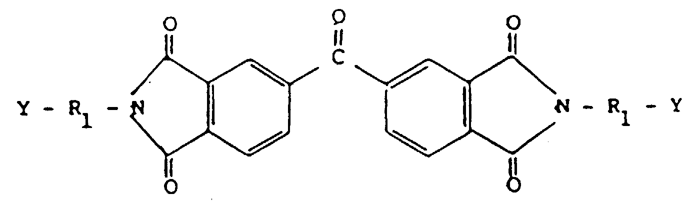
in which R₁ and Y are as defined above, are reacted with compounds of the formula III
R₂ - NH₂
in which R₂ is as defined above, in a diluent which is inert under reaction conditions.

2. Process for the preparation of compounds of the formula I, in which
R₁ denotes a straight-chain alkylene radical having 1 to 4 C atoms or an m- or p-phenylene or benzylidene radical which is optionally substituted by (C₁ to C₄)-alkyl or (C₁ to C₄)-alkoxy, and R₂ denotes the radical OH, a straight-chain, saturated alkyl radical having 1 to 18 C atoms, which can be substituted by OH, NH₂ or COOH, or a radical of the formula NH-CO-NHR₃, wherein R₃ can be hydrogen or a phenyl radical, characterized in that compounds of the formula II 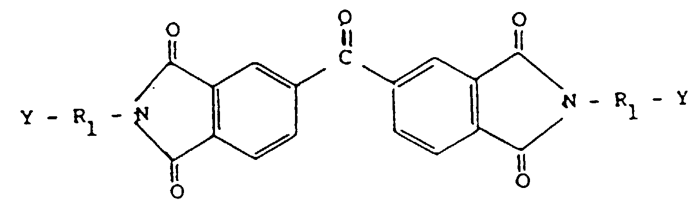 in which R₁ and Y are as defined above, are reacted with compounds of the formula III
R₂ - NH₂
in which R₂ is as defined above, in a diluent which is inert under reaction conditions.

3. Process for the preparation of compounds of the formula I, in which
R₁ denotes an ethylene or p-phenylene radical and R₂ denotes the radical OH, a straight-chain, saturated alkyl radical having 1 to 8 C atoms, which can be substituted by OH, NH₂ or COOH, or a radical of the formula NH-CO-NHR₃, wherein R₃ can be hydrogen or a phenyl radical, characterized in that compounds of the formula II 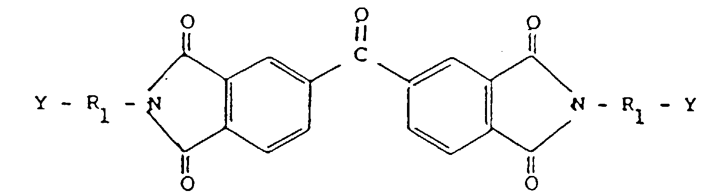 in which R₁ and Y are as defined above, are reacted with compounds of the formula III
R₂ - NH₂
in which R₂ is as defined above, in a diluent which is inert under reaction conditions.

4. Process according to Claims 1 to 3, characterized in that 1 to 10 mol, preferably 1.05 to 5 mol of the compound of the formula III are employed per mol of the compound II.

5. Process for the preparation of polyesters of the formula IVa
⁅CO-Z-CO-O-Ar-O⁆
in which Z denotes a substituted or unsubstituted, straight-chain or branched alkylene radical having 1 to 8 C atoms, or an o-, m- or p-phenylene radical or a radical of the formula V 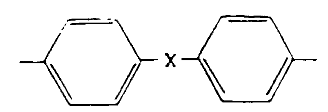 wherein X can be -CH₂-, -C(CH₃)₂-, -O-, -S-, or -SO₂-, and Ar denotes a radical of the formula VI 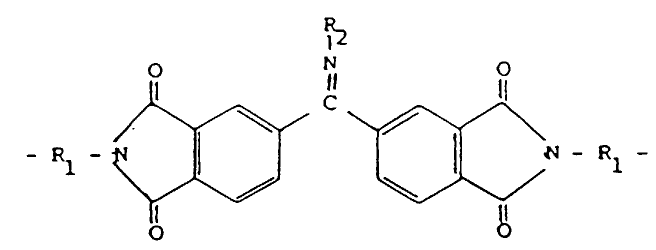 in which R₁ and R₂ are as defined in Claim 1, characterized in that compounds of the formula I in which Y is hydroxyl, and R₁ and R₂ are as defined in Claim 1 are reacted in a diluent which is inert under reaction conditions with dicarboxylic acid dichlorides of the formula VIIa
Cl - CO - Z - CO - Cl
in which Z is as defined above, at temperatures between -30°C and 50°C.

6. Process according to Claim 5, characterized in that compounds of the formula I in which Y is hydroxyl and R₁ and R₂ are as defined in Claim 3, are reacted with dicarboxylic acid dichlorides of the formula VIIa in which Z is an m- or p-phenylene radical and R₁, R₂ and R₃ are as defined in Claim 3.

7. Process for the preparation of polyesters of the formula IV b,
⁅O-Z-O-CO-Ar-CO⁆
in which Z and Ar are as defined in Claim 5, characterized in that compounds of the formula I in which Y is carboxyl and R₁ and R₂ are as defined in Claim 1, are converted into the corresponding dicarboxylic acid dichlorides, anhydrides or esters and are subsequently reacted with diols of the formula VII b
HO - Z - OH
in which Z is as defined in Claim 5, in a diluent which is inert under reaction conditions, at temperatures from -30°C to 50°C.

8. Process according to Claim 7, characterized in that compounds of the formula I in which Y is carboxyl and R₁ and R₂ are as defined in Claim 3, are reacted with diols of the formula VIIb in which Z is an m- or p-phenylene radical and R₁, R₂ and R₃ are as defined in Claim 3.

9. Process for the preparation of polyamides of the formula VIII
⁅NH-Z-NH-CO-Ar-CO⁆
in which Ar and Z are as defined in Claim 5, characterized in that compounds of the formula I in which Y is carboxyl and R₁ and R₂ are as defined in Claim 1, are converted into the corresponding dicarboxylic acid dichloride and are subsequently reacted with a diamine of the formula IX
NH₂ - Z - NH₂
in which Z is as defined in Claim 5, in a diluent which is inert under reaction conditions at temperatures between -30°C and 50°C.

10. Process according to Claim 9, characterized in that compounds of the formula I in which Y is carboxyl and R₁ and R₂ are as defined in Claim 3, are reacted with diamines of the formula IX in which Z is a radical of the formula X 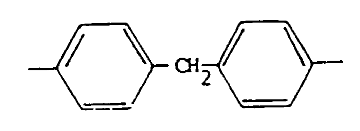 and R₁, R₂ and R₃ are as defined in Claim 3.

## Revendications (Revendications pour l'(les) Etat(s) contractant(s) suivant(s): AT, BE, CH, DE, DK, FR, GB, GR, IT, LI, LU, NL, PT, SE)

1. Composés de formule I 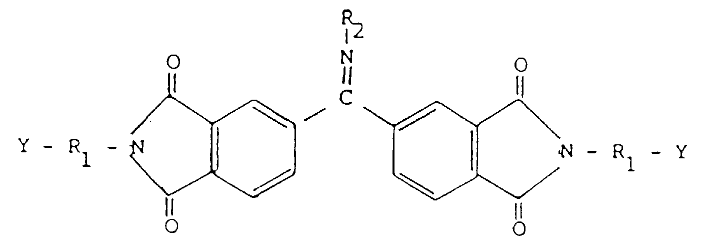 où Y signifie un groupe hydroxyle ou carboxyle,
R₁ signifie un reste alkylène linéaire ou ramifié ayant de 1 à 6 atomes de carbone, ou un reste o-, m- ou p-phénylène ou benzylidène éventuellement substitué par Cl, par un groupe alkyle (en C₁ à C₅) ou par un groupe alcoxy (en C₁ à C₅), et
R₂ signifie le reste OH, un reste alkyle linéaire ou ramifié, saturé ou mono- ou polyinsaturé, ayant de 1 à 20 atomes de carbone, qui peut être substitué par Cl, OH, NH₂, CONH₂ ou COOR₃, ou un reste ayant la formule NH-CO-NHR₃, où
R₃ peut être l'hydrogène, un reste alkyle ayant de 1 à 4 atomes de carbone ou le reste phényle ou benzyle.

2. Composés de formule I selon la revendication 1, où R₁ signifie un reste alkylène linéaire ayant de 1 à 4 atomes de carbone, ou un reste m- ou p-phénylène ou benzylidène éventuellement substitué par un groupe alkyle (en C₁ à C₄) ou par un groupe alcoxy (en C₁ à C₄), et R₂ signifie le reste OH, un reste alkyle linéaire saturé ayant de 1 à 18 atomes de carbone, qui peut être substitué par OH, NH₂ ou COOH, ou un reste répondant à la formule NH-CO-NHR₃, où R₃ peut être l'hydrogène ou le reste phényle.

3. Composés de formule I selon la revendication 1, où R₁ signifie un reste éthylène ou phénylène, et R₂ signifie le reste OH, un reste alkyle linéaire saturé ayant de 1 à 8 atomes de carbone, qui peut être substitué par OH, NH₂ ou COOH, ou un reste répondant à la formule NH-CO-NHR₃, où R₃ peut être l'hydrogène ou le reste phényle.

4. Procédé de préparation de composés de formule I, caractérisé en ce qu'on fait réagir des composés de formule II 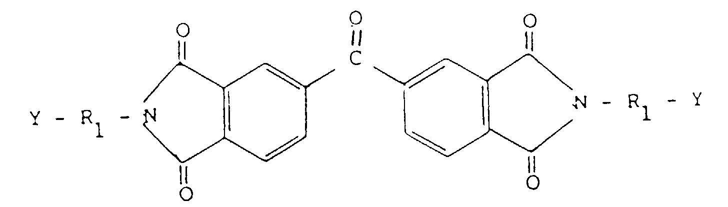 où R₁ et Y sont tels que définis dans la revendication 1, avec des composés de formule III
R₂-NH₂ III
où R₂ est tel que défini dans la revendication 1.

5. Procédé selon la revendication 4, caractérisé en ce qu'on utilise de 1 à 10 moles, de préférence de 1,05 à 5 moles de composé de formule III par mole de composé II.

6. Polyesters de formule IVa
⁅CO-Z-CO-O-Ar-O⁆
ou IVb
⁅O-Z-O-CO-AR-CO⁆
où Z signifie un reste alkylène substitué ou non substitué, linéaire ou ramifié, ayant de 1 à 8 atomes de carbone, ou un reste o-, m- ou p-phénylène ou un reste répondant à la formule V 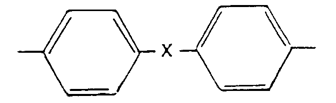 où X peut être -CH₂-, -C(CH₃)₂-, -O-, -S- ou -SO₂-, et Ar signifie un reste répondant à la formule VI 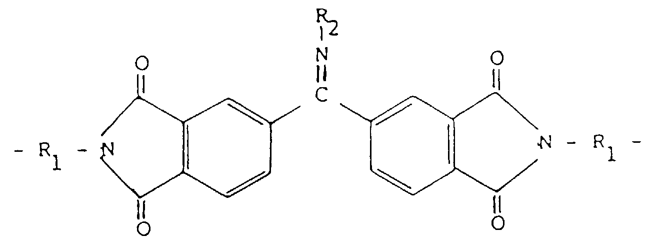 où R₁ et R₂ sont tels que définis dans la revendication 1.

7. Polyesters de formule IVa ou IVb selon la revendication 6, où Z signifie un reste m- ou p-phénylène, et R₁, R₂ et R₃ sont tels que définis dans la revendication 3.

8. Polyamides de formule VII
⁅NH-Z-NH-CO-Ar-CO⁆
où Ar et Z sont tels que définis dans la revendication 6.

9. Polyamides de formule VII selon la revendication 7, où Z signifie un reste répondant à la formule VIII 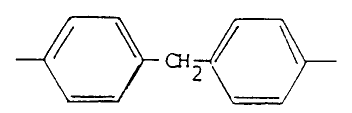 et R₁, R₂ et R₃ sont tels que définis dans la revendication 3.

10. Utilisation des monomères de formule I pour la préparation de polymères.

## Revendications (Revendications pour l'(les) Etat(s) contractant(s) suivant(s): ES)

1. Procédé de préparation de composés de formule I 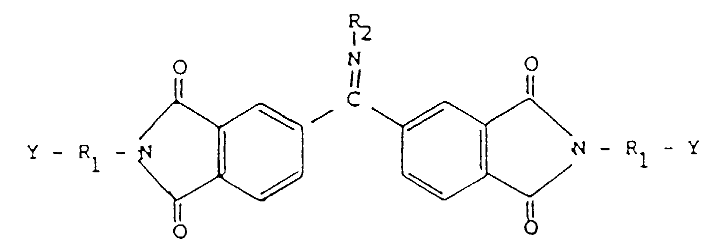 où Y signifie un groupe hydroxyle ou carboxyle,
R₁ signifie un reste alkylène linéaire ou ramifié ayant de 1 à 6 atomes de carbone, ou un reste o-, m- ou p-phénylène ou benzylidène éventuellement substitué par Cl, par un groupe alkyle (en C₁ à C₅) ou par un groupe alcoxy (en C₁ à C₅), et
R₂ signifie le reste OH, un reste alkyle linéaire ou ramifié, saturé ou mono- ou polyinsaturé, ayant de 1 à 20 atomes de carbone, qui peut être substitué par Cl, OH, NH₂, CONH₂ ou COOR₃, ou un reste ayant la formule NH-CO-NHR₃, où
R₃ peut être l'hydrogène, un reste alkyle ayant de 1 à 4 atomes de carbone ou le reste phényle ou benzyle,
caractérisé en ce qu'on fait réagir des composés de formule II 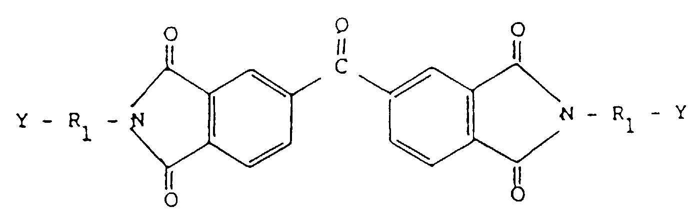 où R₁ et Y sont tels que définis ci-dessus, avec des composés de formule III
R₂-NH₂
où R₂ est tel que défini ci-dessus, dans un diluant inerte dans les conditions de réaction.

2. Procédé de préparation de composés de formule I, où R₁ signifie un reste alkylène linéaire ayant de 1 à 4 atomes de carbone, ou un reste m- ou p-phénylène ou benzylidène éventuellement substitué par un groupe alkyle (en C₁ à C₄) ou par un groupe alcoxy (en C₁ à C₄), et R₂ signifie le reste OH, un reste alkyle linéaire saturé ayant de 1 à 18 atomes de carbone, qui peut être substitué par OH, NH₂ ou COOH, ou un reste répondant à la formule NH-CO-NHR₃, où R₃ peut être l'hydrogène ou le reste phényle, caractérisé en ce qu'on fait réagir des composés de formule II 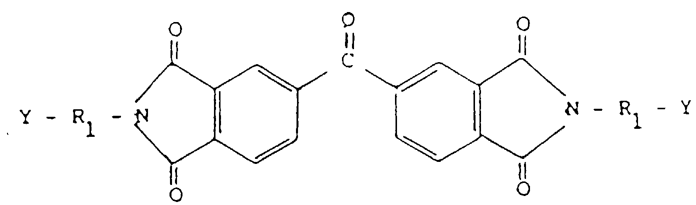 où R₁ et Y sont tels que définis ci-dessus, avec des composés de formule III
R₂-NH₂
où R₂ est tel que défini ci-dessus, dans un diluant inerte dans les conditions de réaction.

3. Procédé de préparation de composés de formule I, où R₁ signifie un reste éthylène ou phénylène, et R₂ signifie le reste OH, un reste alkyle linéaire saturé ayant de 1 à 8 atomes de carbone, qui peut être substitué par OH, NH₂ ou COOH, ou un reste répondant à la formule NH-CO-NHR₃, où R₃ peut être l'hydrogène ou le reste phényle, caractérisé en ce qu'on fait réagir des composés de formule II 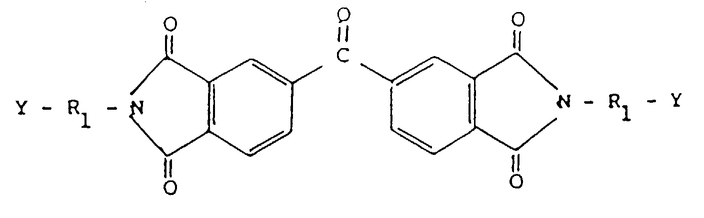 où R₁ et Y sont tels que définis ci-dessus, avec des composés de formule III
R₂-NH₂
où R₂ est tel que défini ci-dessus, dans un diluant inerte dans les conditions de réaction.

4. Procédé selon l'une des revendications 1 à 3, caractérisé en ce qu'on utilise de 1 à 10 moles, de préférence de 1,05 à 5 moles, de composé de formule III par mole de composé II.

5. Procédé de préparation de polyesters de formule IVa
⁅CO-Z-CO-O-Ar-O⁆
où Z signifie un reste alkylène substitué ou non substitué, linéaire ou ramifié, ayant de 1 à 8 atomes de carbone, ou un reste o-, m- ou p-phénylène ou un reste répondant à la formule V 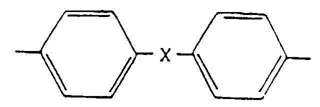 où X peut être -CH₂-, -C(CH₃)₂-, -O-, -S- ou -SO₂-, et Ar signifie un reste répondant à la formule VI 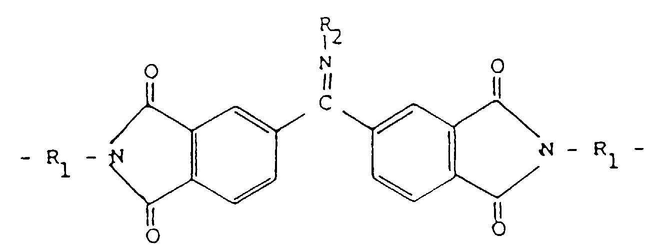 où R₁ et R₂ sont tels que définis dans la revendication 1, caractérisé en ce qu'on fait réagir des composés de formule I, où Y signifie un groupe hydroxyle, et R₁ et R₂ sont tels que définis dans la revendication 1, dans un diluant inerte dans les conditions de réaction, avec des dichlorures d'acide dicarboxylique répondant à la formule VIIa
Cl - CO - Z - CO - Cl
où Z est tel que défini ci-dessus, à des températures comprises entre -30°C et 50°C.

6. Procédé selon la revendication 5, caractérisé en ce qu'on fait réagir des composés de formule I, où Y signifie un groupe hydroxyle, et R₁ et R₂ sont tels que définis dans la revendication 3, avec des dichlorures d'acide dicarboxylique répondant à la formule VIIa, où Z signifie un reste m- ou p-phénylène, et R₁, R₂ et R₃ sont tels que définis dans la revendication 3.

7. Procédé de préparation de polyesters de formule IVb
⁅O-Z-O-CO-Ar-CO⁆
où Z et Ar sont tels que définis dans la revendication 5, caractérisé en ce qu'on transforme des composés de formule I, où Y signifie un groupe carboxyle, et R₁ et R₂ sont tels que définis dans la revendication 1, en dichlorures, anhydrides ou esters d'acide dicarboxylique correspondants, qu'on fait réagir ensuite avec des diols de formule VIIb
HO - Z - OH
où Z est tel que défini dans la revendication 5, dans un diluant inerte dans les conditions de réaction, à des températures comprises entre -30°C et 50°C.

8. Procédé selon la revendication 7, caractérisé en ce qu'on fait réagir des composés de formule I, où Y signifie un groupe carboxyle, et R₁ et R₂ sont tels que définis dans la revendication 3, avec des diols de formule VIIb, où Z signifie un reste m- ou p-phénylène, et R₁, R₂ et R₃ sont tels que définis dans la revendication 3.

9. Procédé de préparation de polyamides de formule VIII
⁅NH-Z-NH-CO-Ar-CO⁆
où Ar et Z sont tels que définis dans la revendication 5, caractérisé en ce qu'on transforme des composés de formule I, où Y signifie un groupe carboxyle, et R₁ et R₂ sont tels que définis dans la revendication 1, en le dichlorure d'acide dicarboxylique correspondant, qu'on fait réagir, ensuite, avec une diamine de formule IX
NH₂ - Z - NH₂
où Z est tel que défini dans la revendication 5, dans un diluant inerte dans les conditions de réaction, à des températures comprises entre -30°C et 50°C.

10. Procédé selon la revendication 9, caractérisé en ce qu'on fait réagir des composés de formule I, où Y signifie un groupe carboxyle, et R₁ et R₂ sont tels que définis dans la revendication 3, avec des diamines de formule IX, où Z signifie un reste de formule X 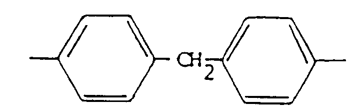 et R₁, R₂ et R₃ sont tels que définis dans la revendication 3.
